(19) Europäisches Patentamt
European Patent Office
Office européen des brevets

(11) Publication number: 0 101 330 B1

# (12) EUROPEAN PATENT SPECIFICATION

(45) Date of publication of patent specification: 24.08.88

(51) Int. Cl.[4]: C 07 D 215/22, C 07 D 215/48, A 61 K 31/47

(21) Application number: 83401198.3

(22) Date of filing: 10.06.83

## (54) 2-Quinolone derivatives.

(30) Priority: 21.06.82 JP 107366/82

(43) Date of publication of application: 22.02.84 Bulletin 84/08

(45) Publication of the grant of the patent: 24.08.88 Bulletin 88/34

(84) Designated Contracting States: CH DE FR GB IT LI

(56) References cited:
Chemical Abstracts vol. 46, no. 11, 10 June 1952, Columbus, Ohio, USA K. TOMITA "The antibacterial properties of compounds containing the tricarbonylmethane group. V.
The syntheses of 3-acyl-2,4-dihydroxyquinolines and of the related compounds with respect to their anti-bacterial properties. column 5044g & J. PharmSoc. Japan vol. 71 1951 pp 1100-112

(73) Proprietor: NIPPON SHINYAKU COMPANY, LIMITED
14, Kisshoin Nishinosho Monguchicho
Minami-ku Kyoto-shi Kyoto (JP)

(72) Inventor: Enomoto, Hiroshi
26-3-7-707 Baba Miba Hashiri
Nagaokakyo-shi Kyoto-fu 617 (JP)
Inventor: Nomura, Tadatoshi
39-905 Okurayama Kobata
Uji-shi Kyoto-fu 611 (JP)
Inventor: Aoyagi, Yoshiaki
8-7-105 Uchidehama
Otsu-shi Shiga-ken 520 (JP)
Inventor: Chokai, Shoichi
1-57 Yamamoto Nakaaraita Sasa-cho
Kameoka-shi Kyoto-fu 621 (JP)
Inventor: Kono, Tatsuhiko
F-306 27 Shinashiyaue
Suita-shi Osaka-fu 565 (JP)
Inventor: Murase, Masao
439-11 Nomuracho
Kusatsu-shi Shiga-ken 525 (JP)
Inventor: Inoue, Kichiro
346-12 Ninomarucho Mukaijima
Fushimi-ku Kyoto 612 (JP)
Inventor: Adachi, Masahiro
6-1 Nagaodai-2-chome
Hirakata-shi Osaka-fu 573-01 (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 101 330 B1

(56) References cited:
Chemical Abstracts vol. 83, no. 21, 24 November 1975, Columbus, Ohio, USA P. VENTURELLA et al. "Reactions between ethyl benzoylacetate and anisidines", page 549, column 1, abstract no. 178773f & J. Heterocycl. Chem., vol. 12, no. 4, 1975, page

Chemical Abstracts vol. 92, no. 17, 28 April 1980, Columbus, Ohio, USA G.M. COPPOLA et al. "The chemistry of 2H-3,1-benzoxazine-2,4(1H)-dione (isatoic anhydride). 7. Reactions with anions of active methylenes to form quinolines", page 572, column 1, abstract no. 146570g& J. Heterocycl çgem. vol. 16, no. 8, 1979, pp 1605-1610

(74) Representative: Hranitzky, Wilhelm Max et al
NOVAPAT - CABINET CHEREAU
5, Place du Molard
CH-1204 Genève (CH)

**Description**

The present invention relates to 2-quinolone derivatives represented by the following general formula (I) and salts thereof as well as manufacture methods therefor and, also, pharmaceuticals consisting of one or more of said compounds as main ingredient(s).

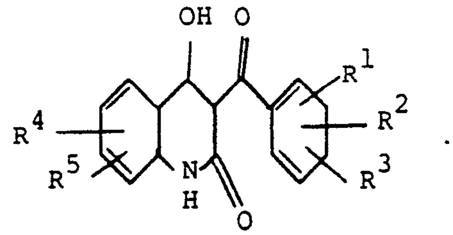

(I)

In the formula, $R^1$, $R^2$, $R^3$, $R^4$ and $R^5$ are same or different and are hydrogen, straight or branched alkyl with one to eight chain length, halogen, straight or branched lower alkoxy with one to five chain length, nitro, cyano, halogenated alkyl (straight or branched) with one to three chain length, or $COOR^6$ (where $R^6$ is hydrogen or straight or branched lower alkyl with one to three chain length excluding the case where all of $R^1$, $R^2$, $R^3$, $R^4$ and $R^5$ are hydrogen and the case of 3-benzoyl-4-hydroxy-7-methoxy-2-quinolone.

The compound 3-benzoyl-4-hydroxy-7-methoxy-2-quinolone is disclosed on page 669 of Journal of Heterocyclic Chemistry, Volume 12, Nr. 4, August 1975. This compound, as such, is thus excluded from the scope of the present invention. However, pharmaceuticals containing 3-benzoyl-4-hydroxy-7-methoxy-2-quinolone as main active ingredient are not excluded since the above-mentioned publication does not disclose any pharmaceutical action of this compound.

Compounds represented by the formula (I), in the case where all of $R^1$, $R^2$, $R^3$, $R^4$ and $R^5$ are hydrogen are mentioned in a paper published in J. Pharm. Soc. Japan *71* (1951), pages 1100 to 1112 under the title "The Antibacterial Properties of Compounds Containing the tricarbonylmethane group V. The syntheses of 3-acyl-2,4-dihydroxyquinolines and of the related compounds with respect to their antibacterial properties".

The use of these compounds as antibacterial agent or in an antibacterial composition is thus suggested by the disclosure made in this paper. This is the reason why these compounds are also excluded from the scope of the present invention.

The compounds of the present invention exhibit antiinflammatory, anti-allergic and expectorant actions and are useful as remedies for allergic diseases and asthma and as antitussives and expectorants.

These compounds exhibit marked characteristics in that they can be administered orally and exhibit long acting action as compared with the compounds of similar conventional type.

For instance, sodium cromoglicate recently developed is reported by Cox, et al. that it is effective for allergic asthma (cf. Advances in Drug Research, volume 5, page 115, 1970). It is supposed that his compound inhibits emission of chemical mediators from mast cells. Unfortunately this compound has a disadvantage that it does not show any pharmaceutical effect by oral route and further that duration of its action is short.

Recently it has been known that SRS—A (slow reacting substance of anaphylaxis) which is one of the chemical mediators plays a main role at the onset of asthma and, under such a situation, development of new and specific pharmaceuticals antagonizing against the action of SRS—A and exhibiting biosynthesis inhibiting action has been expected.

In view of the above, the present inventors have conducted extensive studies in order to find pharmaceuticals satisfying the above requirements and found that the compounds represented by the above formula (I) are substances which exhibit anti-inflammatory action and can be administered orally, exhibit long acting effect, and exhibit strong SRS—A antagonizing action and synthesis inhibiting action, and accomplished the present invention.

In the present invention, lower alkyl means, for example, methyl, ethyl, propyl, isopropyl, butyl, isobutyl, tertiary butyl, pentyl, hexyl, heptyl, and the like. Halogen means, for example, fluorine, chlorine, bromine and iodine. Examples of lower alkoxy are, for example, methoxy, ethoxy, propoxy, isopropoxy, butoxy, tertiary butoxy and the like. Examples of halogenated lower alkyl are, for example, trifluoromethyl and the like.

Representative compounds of the present invention are, for example, as follows:

3-Benzoyl-6-chloro-4-hydroxy-2-quinolone, 3-benzoyl-7-chloro-4-hydroxy-2-quinolone, 3-benzoyl-8-chloro-4-hydroxy-6-methyl-2-quinolone, 3-benzoyl-6,8-dichloro-4-hydroxy-2-quinolone, 3-benzoyl-7-ethoxy-8-nitro-4-hydroxy-2-quinolone, 3-benzoyl-6,7-dimethoxy-4-hydroxy-2-quinolone, 3-benzoyl-4-hydroxy-7-nitro-2-quinolone, 3-benzoyl-6-cyano-4-hydroxy-2-quinolone, methyl 3-benzoyl-4-hydroxy-2-quinolone-5-carboxylate, methyl 3-benzoyl-4-hydroxy-2-quinolone-6-carboxylate, methyl 3-benzoyl-4-hydroxy-2-quinolone-7-carboxylate, methyl 3-benzoyl-4-hydroxy-2-quinolone-8-carboxylate, 3-benzoyl-4-hydroxy-2-quinolone-5-carboxylic acid, 3-benzoyl-4-hydroxy-2-quinolone-6-carboxylic acid, 3-benzoyl-4-hydroxy-2-quinolone-7-carboxylic acid, 3-benzoyl-4-hydroxy-2-quinolone-8-carboxylic acid, 3-benzoyl-4-hydroxy-6-methyl-2-quinolone, 3-benzoyl-4-hydroxy-7-methyl-2-quinolone, 3-benzoyl-4-hydroxy-8-methyl-2-quinolone, 3-benzoyl-6,7-dimethyl-4-hydroxy-2-quinolone, 3-benzoyl-6,8-dimethyl-4-hydroxy-2-quinolone, 3-benzoyl-6-isopropyl-4-hydroxy-2-quinolone, 3-benzoyl-6-n-butyl-4-hydroxy-2-quinolone, 3-benzoyl-6-n-hexyl-4-hydroxy-2-quinolone, 4-hydroxy-3-(2-methoxybenzoyl)-2-quinolone, 3-(2-ethoxybenzoyl)-4-hydroxy-2-quinolone, 4-hydroxy-3-(2-isopropoxybenzoyl)-2-quinolone, 4-hydroxy-3-(4-methoxybenzoyl)-2-quinolone, 3-(2,4-dimethoxybenzoyl)-4-hydroxy-2-quinolone, 3-(3,4-dimethoxybenzoyl)-4-hydroxy-2-quinolone, 3-(3,4-dimethoxybenzoyl)-4-hydroxy-6-methyl-2-quinolone, 3-(3,4-dimethoxybenzoyl)-4-hydroxy-7-nitro-2-quinolone, 3-(3,4-dimethoxybenzoyl)-4-hydroxy-8-methyl-2-quinolone, 4-hydroxy-3-(2-isopropoxy-5-methoxybenzoyl)-2-quinolone, 4-hydroxy-3-(2,3,4-trimethoxybenzoyl)-2-quinolone, 4-hydroxy-3-(3,4,5-trimethoxybenzoyl)-2-quinolone, 4-hydroxy-3-(2,4,5-trimethoxybenzoyl)-2-quinolone, 3-(2-chlorobenzoyl)-4-hydroxy-2-quinolone, 3-(2-chlorobenzoyl)-4-hydroxy-6-isopropyl-2-quinolone, 3-(2-chlorobenzoyl)-6,8-dimethyl-4-hydroxy-2-quinolone, 3-(3-chlorobenzoyl)-4-hydroxy-2-quinolone, 3-(4-chlorobenzoyl)-4-hydroxy-2-quinolone, 3-(2-bromo-4-cyanobenzoyl)-4-hydroxy-2-quinolone, 3-(3,4-dichlorobenzoyl)-4-hydroxy-2-quinolone, 3-(2,5-dichloro-4-methylbenzoyl)-4-hydroxy-2-quinolone, 4-hydroxy-3-(2-methylbenzoyl)-2-quinolone, 4-hydroxy-3-(4-methylbenzoyl)-2-quinolone, 3-(2,4-dimethylbenzoyl)-4-hydroxy-2-quinolone, 4-hydroxy-3-(3-trifluoromethylbenzoyl)-2-quinolone, 4-hydroxy-3-(4-isopropylbenzoyl)-2-quinolone, 4-hydroxy-3-(4-n-pentylbenzoyl)-2-quinolone, 4-hydroxy-3-(4-nitrobenzoyl)-2-quinolone, 4-hydroxy-6-isopropyl-3-(4-nitrobenzoyl)-2-quinolone, 6,8-dimethyl-4-hydroxy-3-(4-nitrobenzoyl)-2-quinolone, 4-hydroxy-3-(4-methoxycarbonylbenzoyl)-2-quinolone, 3-(4-carboxybenzoyl)-4-hydroxy-2-quinolone, 4-hydroxy-6-methyl-3-(2,3,4-trimethoxybenzoyl)-2-quinolone, 4-hydroxy-8-methyl-3-(2,3,4-trimethoxybenzoyl)-2-quinolone, 4-hydroxy-7-nitro-3-(2,3,4-trimethoxybenzoyl)-2-quinolone, and the like.

The compound (I) is represented, for the sake of convenience, as the 4-hydroxy-2-quinolone type, but, of course, it may be also in various tautomeric forms as follows:

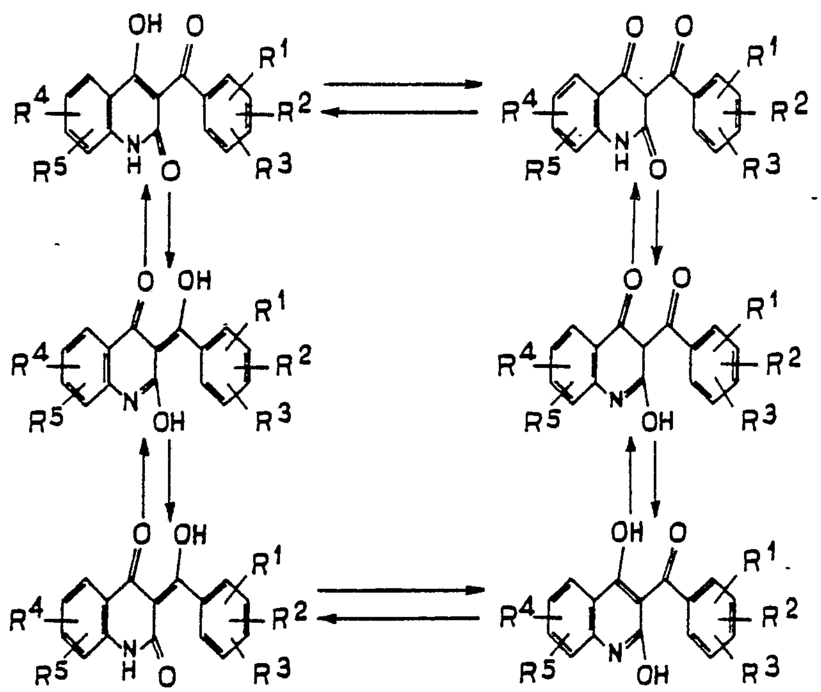

In synthesizing 4-hydroxy-2-quinolone derivatives of the present invention, a compound of the formula (II) may be subjected to a ring closure reaction in the presence of a basic substance.

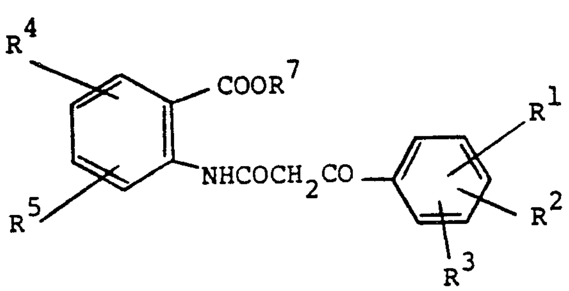

(II)

In the formula, the meanings of $R^1$, $R^2$, $R^3$, $R^4$ and $R^5$ are the same as already defined and $R^7$ stands for lower alkyl such as, for example, methyl, ethyl, propyl, butyl and the like.

The reaction is conducted by heating a compound of the formula (II) 50 to 130°C for one to ten hours in a suitable solvent (for example, methanol, ethanol, benzene, toluene, xylene, tetrahydrofuran, dimethyl formamide and the like) in the presence of a base (for example, metal sodium, sodium hydride, sodium alkoxide, triphenyl methyl sodium, potassium hydroxide, calcium hydroxide and the like).

The compound of the formula (II) is prepared by the reaction of a compound of the formula (III)

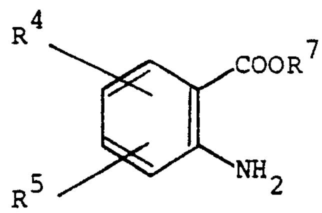

(III)

in which, the meanings of $R^4$, $R^5$ and $R^7$ are same as already defined with a compound of the formula (IV)

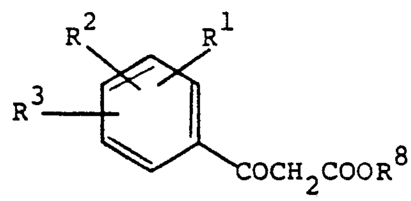

(IV)

in which, the meanings of $R^1$, $R^2$ and $R^3$ are the same as already defined and $R^8$ stands for hydrogen and lower alkyl (for example, methyl, ethyl, propyl, butyl and the like) at 130 to 250°C for one to twenty four hours case when $R^8$ is lower alkyl and when the $R^8$ stands for hydrogen (including the case where the compound is salt), the compound of the formula (II) can be prepared by keeping the compound (III) and the compound (IV) at 0 to 50°C for three to twenty four hours in a suitable solvent (for example, tetrahydrofuran, dimethyl formamide), in the presence of a condensation agent.

Compounds of the present invention may also be prepared by subjecting a compound of the formula (V)

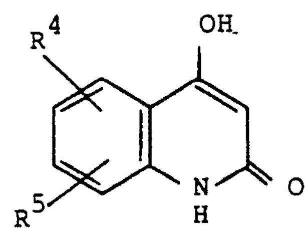

(V)

and the compound of the formula (VI)

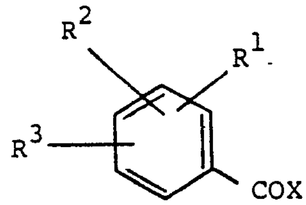

(VI)

(wherein X is halogen)

(in these formulas, meanings of $R^4$, $R^5$, $R^1$, $R^2$ and $R^3$ are the same as already defined) to a Friedel-Crafts reaction at room temperature to 150°C for two to twenty hours in the presence of Lewis acid (such as, for example, aluminum chloride, antimony pentachloride, ferrous chloride, ferric chloride, stannic chloride, zinc chloride, titanium tetrachloride, and the like) in a suitable solvent (such as, for example, nitrobenzene, carbon disulfide, methylene chloride, carbon tetrachloride, 1,2-dichloroethane, and the like).

The compound of the formula (I) may also be prepared by preparing a compound of the formula (VII)

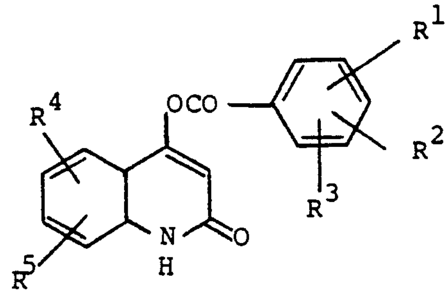

(VII)

in which the meanings of $R^1$, $R^2$, $R^3$, $R^4$ and $R^5$ are the same as already defined from the compounds (V) and (VI) followed by subjecting it to a Fries rearrangement.

4-Hydroxy-2-quinolone compounds of the formula (V) are prepared by heating an N-acetylanthranilate of the formula (VIII)

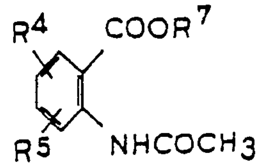

(VIII)

in which, the meanings of $R^4$, $R^5$ and $R^7$ are the same as already defined at 50 to 130°C for one to twelve hours in the presence of a base (for example, metal sodium, sodium hydride, triphenyl methyl sodium, potassium hydroxide, calcium hydroxide and the like) in a suitable solvent (for example, methanol, ethanol, benzene, toluene, xylene, tetrahydrofuran, dimethylformamide).

Alternatively, the compounds of the formula (V) may also be prepared by heating malonic monoanilide of the formula (IX)

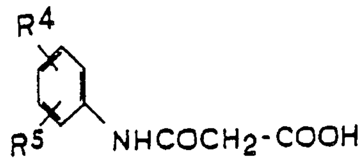

(IX)

(in which, the meanings of $R^4$ and $R^5$ are the same as already defined) at 100 to 200°C for two to twenty four hours in a suitable dehydrating ring closure reagent (such as, for example, polyphosphoric acid, concentrated sulfuric acid and the like).

There are also many other routes for the synthesis of compounds of the formula (V). For example, malonic bianilide is heated at 250 to 350°C in vacuo; anthranilic acid ester is made to react with diketene; anthranilic acid is heated in acetic anhydride; 3-carboxymethylbenzoisoxazole is used as a starting material; 2-methyl-3,1-benzoxazin-4-one is made to react with sodium alkoxide in xylene.

4-Hydroxy-2-quinolone derivatives of the present invention can also be synthesized in a single step by heating benzoylmalonic acid ester with aniline in nitrobenzene.

4-Hydroxy-2-quinolone derivatives prepared by the above methods can easily be isolated and purified by conventional methods such as, for example, recrystallization, chromatography and the like. Thus prepared present invention compounds can be made into salts thereof with conventional pharmaceutically acceptable basic compounds. Examples of said basic compounds are sodium hydroxide, potassium hydroxide, aluminum hydroxide, calcium hydroxide, sodium carbonate, potassium carbonate, sodium bicarbonate, potassium bicarbonate and other inorganic basic compounds as well as morpholine, piperidine, triethylamine and other organic basic substances. Basic amino acids are, of course, included in the basic compounds.

Compounds according to the present invention exhibit anti-allergic action and, accordingly, can be administered to patients suffering from asthma, hay fever (nose allergy), hives and atopic dermatitis. Further they exhibit anti-inflammatory action and, consequently, they can be administered to patients suffering from chronic rheumatic arthritis, pain after operations, ankylosing spondylitis, osteoarthritis of large joints (such as hips, knees and shoulders), acute gouty arthritis, acute inflammation of upper respiratory, toothache and menstrual pains.

The anti-allergic action of compounds of the present invention, particularly as remedies for asthma were evaluated by a passive cutaneous anaphylaxie assay (PCA) in rats and by measuring anti-SRS—A action using ileus of guinea pigs.

Test Method No. 1 (PCA)

(i) Antiserum abundant in homocytotropic antibody is prepared by the same method as Tada and Okumura did (cf. Journal of Immunology, volume 106, page 1002, 1976). Thus, 1 mg (calculated as an amount of protein) of DNP—As (2,4-dinitrophenyl-coupled ascaris extract) prepared by methods of Strejan and Campbell (Journal of Immunology, volume 98, page 893, 1971) and of Eisen (Journal of American Chemical Society, volume 85, page 4593, 1953) and $1 \times 10^{10}$ pertussis vaccine are administered to each paw of Wistar strain rats (180 to 200 grams body weights) by dividing the dose by four. Five days later, 0.5 mg of DNP—As is administered into back muscle. Eight days after the initial immunization, blood is taken from descending aorta whilst anesthetizing with ether, the resulting serum is stored at −80° and is melted before use.

(ii) The action of the tested compounds is investigated as follows:

Anti-serum obtained by the method (i) is diluted with physiological saline solution double by double successively and 0.05 ml of each diluted solution is administered subcutaneously into the back of Wister strain rats (140 to 160 grams body weight). After 72 hours, a solution of 2 mg (calculated as protein) of

DNP—As and 2.5 mg of Evans Blue dissolved in 1 ml of physiological saline solution is administered intravenously at a dose of 5 ml/kg. 30 minutes after antigen solution administration, the animals are killed and the diameter of blue spots which appeared at the place where antiserum is administered are measured. The PCA test is conducted by the same method as already described using a diluted solution of antiserum which always show 10 mm or more of spot diameters and the effect of the test compounds is judged. Thus antiserum diluted solutions are administered to two places in the back. Test compounds are administered orally at a dose of 10 mg/kg one hour before administration of the antigen solution. From the skin of reacted parts of killed animals leaked or emitted dyestuff is extracted and the amount of the dyestuff is measured. The inhibition ratio is calculated by the following expression:

$$\text{Inhibition Ratio} = (1 - \frac{A'}{A}) \times 100$$

in which A' is an amount of dyestuff in the group treated with the test compounds and A is that in the control group.

Test Method No. 2

Anti-SRS—As action (An anti-action against slow reacting substance of anaphylaxis).

Hartley strain male guinea pigs (300 to 350 grams body weight) are killed and 1.0 to 1.5 cm of ileus is immediately excised from ileocecal parts and is suspended in 10 ml of Tyrode solution (95% $O_2$ — 5% $CO_2$ saturation) containing $10^{-7}$ g/ml of atropine and $10^{-6}$ of pyrilamine. SRS—A (20 units) (the amount of SRS—A showing the same shrinkage as 5 ng of histamine is defined as 1 unit) prepared by using sensitized guinea pig lung is given to cause shrinkage there. Then antagonistic action of test compounds treated five minutes ago against the shrinkage is measured and recorded via isotonic transducer.

$$\text{Inhibition Ratio of Test Compd (\%)} = (1 - \frac{A'}{A}) \times 100$$

in which A' is a height of shrinkage of SRS—A + test compound and A is that of SRS—A.

TABLE 1

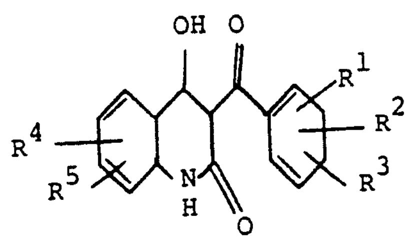

(I)

| Example No. | $R^1$, $R^2$, $R^3$ | $R^4$, $R^5$ | PCA Inhibition Ratio % | $10^{-6}$M SRS—A Inhibition Ratio % |
|---|---|---|---|---|
| 8 | H | 7-Cl | 16.5 | 26.2 |
| 10 | H | 7-$NO_2$ | 33.4 | 22.3 |
| 3 | H | 5'-COOMe | 26.9 | 26.4 |
| 13 | H | 8-COOMe | 25.7 | 19.8 |
| 16 | H | 8-COOH | 30.9 | 22.4 |
| 17 | H | 6-Me | 35.4 | 21.9 |
| 18 | H | 7-Me | 26.0 | 21.3 |
| 19 | H | 8-Me | 29.6 | 19.0 |
| 20 | H | 6,7-$(Me)_2$ | 17.4 | 23.5 |

TABLE 1 (continued)

| Example No. | $R^1$, $R^2$, $R^3$ | $R^4$, $R^5$ | PCA Inhibition Ratio % | $10^{-6}$M SRS—A Inhibition Ratio % |
|---|---|---|---|---|
| 22 | H | 6-n-$C_4H_9$ | 17.4 | 39.5 |
| 23 | H | 6-n-$C_6H_{13}$ | 13.1 | 83.3 (32.8) |
| 24 | 2′-OMe | H | 24.7 | 20.2 |
| 28 | 2′,4′-$(OMe)_2$ | H | 21.2 | 42.9 |
| 2 | 3′,4′-$(OMe)_2$ | H | 25.5 | 24.2 |
| 29 | 3′,4′-$(OMe)_2$ | 6-Me | 12.1 | 42.1 |
| 30 | 3′,4′-$(OMe)_2$ | 7-$NO_2$ | 18.7 | 26.7 |
| 31 | 3′,4′-$(OMe)_2$ | 8-Me | 20.4 | 20.5 |
| 32 | 2′,3′,4′-$(OMe)_3$ | H | 16.3 | 100 (83.3) |
| 35 | 2′-Cl | H | 45.6 | 32.6 |
| 36 | 2′-Cl | 6-i-$C_3H_7$ | 12.4 | 60.0 |
| 37 | 2′-Cl | 6,8-$(Me)_2$ | 20.8 | 28.3 |
| 39 | 4′-Cl | H | 19.8 | 27.0 |
| 46 | 4′-n-$C_5H_{11}$ | H | 3.2 | 26.9 |
| 47 | 4′-$NO_2$ | H | 49.4 | 22.0 |
| 48 | 4′-$NO_2$ | 6-i-$C_3H_7$ | 8.6 | 29.8 |
| 51 | 4′-COOMe | H | 7.4 | 50.0 |
| 7 (Sodium Salt) | H | 6-n-hexyl | 15.2 | 91.2 |
| 55 (Calcium Salt) | H | 6-n-hexyl | 13.6 | 85.6 |

The value in (   ) is for $10^{-7}$M

The antiinflammatory action of the compounds of the present invention was evaluated by the following method.

Carrageenin edema in hind foot of rats.

Experimental method:

SD-strain rats (150 grams body weight in average) are used, one group consisting of five rats. 0.5% Carrageenin solution (0.1 ml) dissolved in physiological saline water was injected hypodermically to right hind foot paw of rats. Test compound was given orally (200 mg/kg) one hour before the carrageenin treatment. Foot volumes before and three hours after the carrageenin treatment were measured, the difference thereof was compared with that of the control group and the data were used as a target of the pharmaceutical effect.

Representative results are as follows:

| Example Number | % Inhibition |
|---|---|
| 1 | 42.1 |
| 18.1 | 22.0 |
| 19 | 70.0 |
| 3 | 32.8 |
| 43 | 15.7 |
| 44 | 15.9 |
| 5 | 32.1 |
| 30 | 21.3 |
| 48 | 12.5 |
| 31 | 77.5 |
| 23 | 15.9 |
| 54 | 13.1 |
| 59 | 12.8 |
| 55 | 10.0 |
| Acetylsalicylic Acid | 31.6 |

Acute toxicity is observed by oral administration of 400 mg/kg of compounds of the present invention to male mice for two weeks. One group consists of four mice and the lethal numbers of mice are given in the numerator. The lethal ratio of all other compounds are all 0/4 by administration of 200 mg/kg.

| Example Number | Lethal Ratio |
|---|---|
| 10 | 0/4 |
| 13 | 1/4 |
| 16 | 0/4 |
| 19 | 0/4 |
| 23 | 0/4 |
| 32 | 0/4 |
| 35 | 0/4 |
| 47 | 0/4 |

The mode of administration of the present invention compounds is generally as follows. In oral administration, once to thrice daily and 1 to 1000 mg each; in rectal administration, once to thrice daily and 1 to 500 mg each; in inhalation, twice to thrice to bronchi, 0.1 to 100 mg each; in intravenous administration, three to four times daily and 0.1 to 50 mg each; in nasal administration, twice to thrice daily and 0.1 to 100 mg each; as eye drop, three to four times daily and 0.1 to 50 mg each; as ointment, twice to thrice daily and 1 to 100 mg each.

Compounds of the present invention can be applied to preparatory compositions by the following methods. Such preparatory compositions can be practically used using, if desired, a suitable

pharmaceutical carrier or bulking agent, by conventional route. It is desired that those compositions are offered as a form adequate for being absorbed from the stomach and intestinal canals. Still it is all right to administer them by non-oral route.

Examples of unit dose administration form are tablets, powders, fine particles, pills, granules and capsules. They may contain conventional vehicles or diluents such as binders (such as sirup, gum arabicum, gelatin, sorbitol, tragacanth, polyvinyl pyrrolidone, polyvinyl alcohol, hydroxypropyl methyl cellulose, hydroxypropyl cellulose, methyl cellulose, carboxymethyl cellulose sodium, and the like), diluents (such as lactose, sucrose, corn starch, calcium phosphate, sorbitol, crystalline cellulose and the like), lubricants (such as magnesium stearate, talc, polyethyleneglycol, silica and the like), disintegrating agents (such as potato starch, hydroxypropyl cellulose with lower degree of substitution, microcrystalline cellulose and the like), acceptable wetting agents (such as sodium laurylsulfate and the like), and the like. Tablets may be subjected to coating by a known method.

Liquid preparations may be aqueous or oil suspensions, solutions, sirups, elixiers and the like and may be dried product which is redissolved in suitable vehicles such as water before use. Those liquidal preparations may contain conventional additives. They are, for example, suspending agents such as methylcellulose, carboxymethylcellulose sodium, hydroxypropyl cellulose, hydroxypropyl methyl cellulose, polyvinyl pyrrolidone, polyvinyl alcohol, acaciá, tragacanth, gelatin, sodium alginate and the like; emulsifiers such as lecithin, sorbitan, fatty acid esters, gum arabicum, tragacanth and the like; wetting agents such as polyoxyethylene sorbitan fatty acid esters, polyoxyethylene fatty acid esters, polyoxyethylene derivatives of hydrogenated castor oil and the like; non-aqueous vehicles such as sesami oil, soybean oil and other plant oils, propylene glycol, polyethylene glycol, ethyl alcohol and the like; antiseptics such as methyl p-hydroxybenzoate, propyl p-hydroxybenzoate, sorbic acid and the like; sweetening agents such as simple sirup, sucrose, sorbitol, mannitol.

Bases for rectal administration, fatty or oily bases such as cacao fat, triglyceride (Widepsol — Registered Trademark), and the like or water-soluble bases such as polyethylene glycol and the like can be used. The so-called rectal capsules in which the active constituent is suspended in vegetable oil and made into gelatin capsules may also be used.

Those preparations may be made into long acting preparations by known methods and forms or may be made into microcapsules.

It is desired that about 0.1 to 99% (or 0.5 to 90% in general) of one or more of the present invention effective compounds is contained in total compositions in the preparations.

Examples of manufacturing pharmaceutical preparations containing the present invention compounds as main constituents are given as hereunder.

Examples of Manufacturing Pharmaceutical Preparations:

(1) Capsules mainly composed of 4-hydroxy-3-(2,3,4-trimethoxy-benzoyl)-2-quinolone (compound of Example No. 32).

The compound of the Example 32 is mixed uniformly with diluents as per the following ratios and filled in hard gelatin capsules to give the desired preparation.

| | |
|---|---|
| Compounds of Example 32 | 50 mg |
| Hydroxypropyl cellulose of lower degree of substitution | 20 mg |
| Lactose | 84 mg |
| Starch | 40 mg |
| Talc | 5 mg |
| Magnesium stearate | 1 mg |
| Mixed to make | 200 mg per capsule |

(2) Tablets containing 3-(2-chlorobenzoyl)-4-hydroxy-2-quinolone (compound of Example 35) as a main constituent.

Pulverized compound of Example 35 (100 mg) is mixed with 100 mg of lactose, 75 mg of crystalline cellulose and 40 mg of potato starch, kneaded with a binder solution prepared from 10 mg of polyvinyl alcohol, the mixture is passed through a sieve of 16 mesh, the resulting granules are dried, and once again passed through a sieve of 16 mesh. Then the granules are mixed with 3 mg of magnesium stearate and 7 mg of talc and compressed into tablets. The resulting tablets are, if necessary, coated with conventional coating base or with sucrose.

The present invention is further illustrated by the following examples.

Example 1

3-Benzoyl-6-chloro-4-hydroxy-2-quinolone

To 6.0 grams of ethyl 2-amino-5-chlorobenzoate is added 6.4 grams of ethyl benzoylacetate, the mixture is heated at 180 to 190°C for ten hours, cooled, crystals separated out therefrom are collected by filtration, and washed with methanol to give 5.1 grams of ethyl 2-benzoylacetylamino-5-chlorobenzoate, colorless needles. To 200 ml of ethanol is added 640 mg of metal sodium, then 4.8 grams of ethyl 2-benzoylacetylamino-5-chlorobenzoate is added thereto, the mixture is heated to reflux for two hours, cooled, poured into ice water, adjusted to pH 3 with 10% hydrochloric acid, crystals separated out therefrom are collected by filtration, and recrystallized from dimethylformamide and methanol to give 1.9 grams of 3-benzoyl-6-chloro-4-hydroxy-2-quinolone, pale yellow needles, melting at above 300°C.

Elementary analysis calculated for $C_{16}H_{10}ClNO_3$: C 63.12, H 3.36, N 4.67; Found: C 64.38, H 3.14, N 4.59.

Example 2

3-(3,4-Dimethoxybenzoyl)-4-hydroxy-2-quinolone

Ethyl anthranilate (1.44 grams) and 2.2 grams of ethyl 3,4-dimethoxybenzoylacetate are dissolved in 100 ml of xylene and heated to reflux for five hours with 200 mg of sodium ethoxide. After cooled, crystals separated out therefrom are collected by filtration and recrystallized from methanol to give 0.5 gram of 3-(3,4-dimethoxybenzoyl)-4-hydroxy-2-quinolone, pale yellow needles, melting point 235 to 236°C.

Elementary analysis calculated for $C_{18}H_{15}NO_5$: C 66.45, H 4.65, N 4.31; Found: C 66.17, H 4.69, N 4.07.

Example 3

Methyl 3-benzoyl-4-hydroxy-2-quinolone-5-carboxylate

A mixture of 2.1 grams of dimethyl 3-aminophthalate, 1.4 grams of benzoylacetic acid and 2.9 grams of dicyclohexylcarbodiimide is dissolved in 15 ml of tetrahydrofuran and the mixture is stirred at room temperature for eighteen hours. The insoluble matter are removed by filtration, the filtrate is concentrated, and 1.9 grams of dimethyl 3-benzoylacetylaminophthalate, colorless crystals, melting point 100 to 103°C, is obtained. This (1.9 grams) is dissolved in 30 ml of methanol, heated to reflux for four hours with 0.48 gram of sodium hydride, cooled, adjusted to pH 3 with diluted hydrochloric acid, crystals separated out therefrom are collected by filtration and recrystallized from methanol to give 1.3 grams of methyl 3-benzoyl-4-hydroxy-2-quinolone-5-carboxylate, pale yellow needles, melting point 263 to 265°C.

Elementary analysis calculated for $C_{18}H_{13}NO_5$: C 66.87, H 4.05, N 4.33; Found: C 66.99, H 3.80, N 4.03.

Example 4

3-Benzoyl-4-hydroxy-2-quinolone-5-carboxylic acid

To 0.6 gram of methyl 3-benzoyl-4-hydroxy-2-quinolone-5-carboxylate are added 1.5 grams of sodium hydroxide, 30 ml of water and 30 ml of dimethyl sulfoxide, the mixture is heated at 100 to 120°C for three hours with stirring, cooled, acidified with diluted hydrochloric acid, and crystals separated out therefrom are collected by filtration to give 0.54 gram of 3-benzoyl-4-hydroxy-2-quinolone-5-carboxylic acid, colorless powder, melting at above 300°C.

Elementary analysis calculated for $C_{17}H_{11}NO_5$: C 66.02, H 3.59, N 4.53; Found: C 65.78, H 3.47, N 4.41.

Example 5

3-Benzoyl-4-hydroxy-6-isopropyl-2-quinolone

(a) p-Isopropylaniline (13.5 grams) and 100 grams of diethyl malonate are heated at 175 to 180°C for two hours, an excess of diethyl malonate is removed in vacuo, to the residue are added 8 grams of potassium hydroxide and 300 ml of methanol, and the mixture is heated to reflux for three hours. After evaporation of methanol, the residue is dissolved in water, the solution is acidified with diluted hydrochloric acid, and crystals separated out therefrom are collected by filtration. The resulting crystals (16.3 grams) are heated in 100 grams of polyphosphoric acid at 160°C for four hours, poured into ice water, crystals separated out therefrom are collected by filtration, and washed with water to give 11 grams of 4-hydroxy-6-isopropyl-2-quinolone, pale brown crystals, melting at above 300°C.

To 10 ml of nitrobenzene is added 3.3 grams of anhydrous aluminum chloride, benzoyl chloride is dropped thereinto with stirring, to the resulting uniform solution is added 2.5 grams of 4-hydroxy-6-isoproyl-2-quinolone, the mixture is heated with stirring at 110°C for eight hours, then concentrated hydrochloric acid is added to make the reaction solution decomposed, water is added thereto, crystals separated out therefrom are collected by filtration, washed with ether and methanol, and recrystallized from chloroform and methanol to give 1.6 grams of 3-benzoyl-4-hydroxy-6-isopropyl-2-quinolone, pale yellow needles, melting point 270°C.

Elementary analysis calculated for $C_{19}H_{17}NO_3$: C 74.25, H 5.58, N 4.56; Found: C 74.38, H 5.51, N 4.61.

(b) To 2.5 grams of 4-hydroxy-6-isopropyl-2-quinolone was added 50 ml of dimethyl formamide, then 600 mg of 50% sodium hydride was added with stirring, the mixture was stirred for 30 minutes, and 1.73 grams of benzoyl chloride was dropped therein. The mixture was stirred at room temperature for one hour, poured over into ice water, and extracted with chloroform. The solvent was evaporated from the extract, a suitable amount of a Lewis acid catalyst and 10 ml of nitrobenzene was added to the residue, and the mixture was heated on a steam bath for 4 hours. To this was added concentrated hydrochloric acid to

decompose the reaction solution, then water was added thereto, and crystals separated out therefrom were collected by filtration. The crystals were washed with ether and methanol and recrystallized from methanol and chloroform to give 1 gram of 3-benzoyl-4-hydroxy-6-isopropyl-2-quinolone pale yellow needles, m.p. 270°C.

### Example 6

3-Benzoyl-6,8-dichloro-4-hydroxy-2-quinolone

Ethyl 2-amino-3,5-dichlorobenzoate (4.0 grams) is dissolved in 100 ml of chloroform, 2.5 ml of pyridine is added, the mixture is heated to reflux for twenty four hours with 2.5 ml of acetyl chloride, the solvent is evaporated therefrom, n-hexane is added thereto, and colorless crystals are obtained. The resulting crystals (3.5 grams) is heated to reflux for five hours with 100 ml of toluene and 610 mg of sodium hydride, cooled, 100 ml of water is added thereto, an aqueous layer is separated therefrom, acidified with diluted hydrochloric acid, and crystals separated out therefrom are collected by filtration to give 1.5 grams of 6,8-dichloro-4-hydroxy-2-quinolone, pale yellow powder, melting at above 300°C.

Benzoyl chloride (0.93 gram) is added to a mixture of 1.5 grams of anhydrous aluminum chloride and 10 ml of nitrobenzene, the mixture is stirred at room temperature until it becomes uniform, stirred at 105 to 110°C for eight hours with 1.26 grams of 6,8-dichloro-4-hydroxy-2-quinolone, decomposed with concentrated hydrochloric acid, small amount of water is added thereto, and crystals separated out therefrom are collected by filtration. The crystals are washed with water and then washed with methanol and ether to give 1.2 grams of 3-benzoyl-6,8-dichloro-4-hydroxy-2-quinolone, pale yellow powder, melting point 258 to 259°C.

Elementary analysis calculated for $C_{16}H_9Cl_2NO_3$: C 57.51, H 2.71, N 4.19; Found: C 57.31, H 2.64, N 4.11.

### Example 7

3-Benzoyl-6-n-hexyl-4-hydroxy-2-quinolone sodium salt.

Sodium hydroxide (560 mg) is dissolved in 100 ml of methanol, the mixture is heated with stirring with 5.5 grams of 3-benzoyl-6-n-hexyl-4-hydroxy-2-quinolone, cooled, insoluble matters are removed by filtration, the filtrate is concentrated and evaporated to dryness, and the residue is recrystallized from isopropyl alcohol to give 5 grams of sodium salt of 3-benzoyl-6-n-hexyl-4-hydroxy-2-quinolone, yellow crystals, melting point 217 to 221°C.

Elementary analysis calculated for $C_{22}H_{22}NO_3Na$: C 71.15, H 5.97, N 3.77; Found C 70.87, H 5.99, N 3.68.

By applying methods for the manufacture of Examples 1 to 7, compounds of Examples 8 to 63 are obtained.

They are given in the following tables.

| Example No. | $R^1$, $R^2$, $R^3$ | $R^4$, $R^5$ | Appearance | M.p. (°C) | Experimental Formula | Elementary Analysis (%) Upper column: Calcd. Lower column: Found |
|---|---|---|---|---|---|---|
| 8 | H | 7-Cl | Colorless needles | >300 | $C_{16}H_{10}ClNO_3$ | C 64.12 H 3.36 N 4.67<br>C 64.34 N 3.08 N 4.51 |
| 9 | H | 6,7-(OMe)$_2$ | Pale yellow powder | >300 | $C_{18}H_{15}NO_5$ | C 66.45 H 4.65 N 4.31<br>C 66.66 H 4.45 N 4.26 |
| 10 | H | 7-$NO_2$ | Yellow needles | >300 | $C_{16}H_{10}N_2O_5$ | C 61.94 H 3.25 N 9.03<br>C 61.91 H 2.96 N 8.84 |
| 11 | H | 6-COOMe | Colorless powder | 279~281 | $C_{18}H_{13}NO_5$ | C 66.87 H 4.05 N 4.33<br>C 66.65 H 3.76 N 4.13 |
| 12 | H | 7-COOMe | Pale yellow powder | 285~287 | $C_{18}H_{13}NO_5$ | C 66.87 H 4.05 N 4.33<br>C 66.83 H 3.83 N 4.43 |
| 13 | H | 8-COOMe | " | 184~186 | $C_{18}H_{13}NO_5$ | C 66.87 H 4.05 N 4.33<br>C 66.72 H 4.02 N 4.20 |
| 14 | H | 6-COOH | Colorless powder | >300 | $C_{17}H_{11}NO_5$ | C 66.02 H 3.59 N 4.53<br>C 66.09 H 3.33 N 4.48 |
| 15 | H | 7-COOH | " | >300 | $C_{17}H_{11}NO_5$ | C 66.02 H 3.59 N 4.53<br>C 65.95 H 3.35 N 4.57 |
| 16 | H | 8-COOH | Pale yellow powder | 286~288 | $C_{17}H_{11}NO_5$ | C 66.02 H 3.59 N 4.53<br>C 65.73 H 3.58 N 4.45 |
| 17 | H | 6-Me | Yellow needles | 295~296 | $C_{17}H_{13}NO_3$ | C 73.11 H 4.68 N 5.02<br>C 73.37 H 4.53 N 4.97 |

| Example No. | $R^1$, $R^2$, $R^3$ | $R^4$, $R^5$ | Appearance | M.p. (°C) | Experimental Formula | Elementary Analysis (%) Upper column: Calcd. Lower column: Found |
|---|---|---|---|---|---|---|
| 18 | H | 7-Me | " | >300 | $C_{17}H_{13}NO_3$ | C 73.11 H 4.68 N 5.02<br>C 73.35 H 4.52 N 4.93 |
| 19 | H | 8-Me | Pale yellow needles | 273~274 | $C_{17}H_{13}NO_3$ | C 73.11 H 4.68 N 5.02<br>C 73.09 H 4.49 N 5.19 |
| 20 | H | 6,7-$(Me)_2$ | Pale yellow powder | 292 | $C_{18}H_{15}NO_3$ | C 73.70 H 5.15 N 4.78<br>C 73.58 H 4.97 N 4.49 |
| 21 | H | 6,8-$(ME)_2$ | Pale yellow needles | >300 | $C_{12}H_{15}NO_3$ | C 73.70 H 5.15 N 4.78<br>C 73.41 H 4.99 N 4.66 |
| 22 | H | 6-n-$C_4H_9$ | " | 230~231 | $C_{20}H_{19}NO_3$ | C 74.74 H 5.96 N 4.36<br>C 74.91 H 6.04 N 4.35 |
| 23 | H | 6-n-$C_{10}H_{13}$ | " | 206~207 | $C_{22}H_{23}NO_3$ | C 75.62 H 6.63 N 4.01<br>C 75.72 H 6.59 N 4.04 |
| 24 | 2'-OMe | H | Yellow powder | 275~276 | $C_{17}H_{13}NO_4$ | C 69.14 H 4.44 N 4.74<br>C 69.40 H 4.14 N 4.65 |
| 25 | 2'-OEt | H | Colorless powder | 265~267 | $C_{18}H_{15}NO_4$ | C 69.89 H 4.89 N 4.53<br>C 70.08 H 4.82 N 4.66 |
| 26 | 2'-O-i-$C_3H_7$ | H | Colorless powder | 260~262 | $C_{19}H_{17}NO_4$ | C 70.57 H 5.30 N 4.33<br>C 70.52 H 5.36 N 4.26 |
| 27 | 4'-OMe | H | Pale yellow powder | 275~280 | $C_{17}H_{13}NO_4$ | C 69.14 H 4.44 N 4.74<br>C 68.84 H 4.02 N 4.46 |
| 28 | 2',4'-$(OMe)_2$ | H | " | 260~262 | $C_{18}H_{15}NO_5$ | C 66.45 H 4.65 N 4.31<br>C 66.60 H 4.59 N 4.15 |

| Example No. | $R^1$, $R^2$, $R^3$ | $R^4$, $R^5$ | Appearance | M.p. (°C) | Experimental Formula | Elementary Analysis (%) Upper column: Calcd. Lower column: Found |
|---|---|---|---|---|---|---|
| 29 | 3′,4′-$(OMe)_2$ | 6-Me | " | 282~284 | $C_{19}H_{17}NO_5$ | C 67.25 H 5.05 N 4.13<br>C 67.43 H 4.92 N 4.13 |
| 30 | 3′,4′-$(OMe)_2$ | 7-$NO_2$ | Yellow powder | 285~290 | $C_{18}H_{14}N_2O_7$ | C 58.38 N 3.81 N 7.57<br>C 58.09 H 4.18 N 7.33 |
| 31 | 3′,4′-$(OMe)_2$ | 8-Me | Colorless powder | 259~261 | $C_{19}H_{17}NO_5$ | C 67.25 H 5.05 N 4.13<br>C 67.49 H 4.95 N 3.96 |
| 32 | 2′,3′,4′-$(OME)_3$ | H | Pale yellow crystals | 247~249 | $C_{19}H_{17}NO_6$ | C 64.22 H 4.82 N 3.94<br>C 64.50 H 4.64 N 4.10 |
| 33 | 3′,4′,5′-$(OMe)_3$ | H | Colorless needles | 199~200 | $C_{19}H_{17}NO_5$ | C 64.22 H 4.82 N 3.94<br>C 64.26 H 4.90 N 3.79 |
| 34 | 2′,4′,5′-$(OMe)_3$ | H | Yellow needles | 228~230 | $C_{19}H_{17}NO_6$ | C 64.22 N 4.82 H 3.94<br>C 64.47 H 4.71 N 3.81 |
| 35 | 2′-Cl | H | Colorless powder | >300 | $C_{16}H_{10}ClNO_3$ | C 64.12 H 3.34 N 4.68<br>C 64.40 H 3.18 N 4.54 |
| 36 | 2′-Cl | 6-i-$C_3H_7$ | Pale yellow needles | 224~225 | $C_{19}H_{15}ClNO_3$ | C 66.77 H 4.72 N 4.10<br>C 66.97 H 4.61 N 4.14 |
| 37 | 2′-Cl | 6,8-$(Me)_2$ | Pale yellow needles | >300 | $C_{18}H_{14}ClNO_3$ | C 65.96 H 4.31 N 4.27<br>C 66.18 H 4.23 N 4.09 |
| 38 | 3′-Cl | H | Pale yellow needles | 268 | $C_{16}H_{10}ClNO_3$ | C 64.12 H 3.36 N 4.67<br>C 64.33 H 3.18 N 4.53 |
| 39 | 4′-Cl | H | Colorless powder | >300 | $C_{15}H_{10}ClNO_3$ | C 64.12 H 3.36 N 4.67<br>C 64.06 H 3.12 N 4.69 |

| Example No. | $R^1$, $R^2$, $R^3$ | $R^4$, $R^5$ | Appearance | M.p. (°C) | Experimental Formula | Elementary Analysis (%) Upper column: Calcd. Lower column: Found |
|---|---|---|---|---|---|---|
| 40 | 3',4'-$(Cl)_2$ | H | Yellow powder | 285~290 | $C_{16}H_9Cl_2NO_3$ | C 57.51 H 2.71 N 4.19<br>C 57.61 H 2.41 N 3.91 |
| 41 | 2'-Me | H | Colorless powder | 292~295 | $C_{17}H_{13}NO_3$ | C 73.11 H 4.69 N 5.02<br>C 72.92 H 4.44 N 5.00 |
| 42 | 4'-Me | H | Pale yellow needles | 290~292 | $C_{17}H_{13}NO_3$ | C 73.11 H 4.69 H 5.02<br>C 73.19 H 4.43 N 5.02 |
| 43 | 2',4'-$(Me)_2$ | H | Colorless powder | 253~255 | $C_{18}H_5NO_3$ | C 73.70 H 5.15 N 4.78<br>C 73.87 N 5.08 N 4.71 |
| 44 | 3'-$CF_3$ | H | " | 258~260 | $C_{17}H_{10}F_3NO_3$ | C 61.27 H 3.02 N 4.20<br>C 61.55 H 2.87 N 4.05 |
| 45 | 4'-i-$C_3H_7$ | H | " | 286~288 | $C_{19}H_{17}NO_3$ | C 74.25 H 5.58 N 4.56<br>C 74.45 H 5.33 N 4.27 |
| 46 | 4'-n-$C_5H_{11}$ | H | Pale brown powder | 199~202 | $C_{20}H_{21}NO_3$ | C 74.28 N 6.55 N 4.33<br>C 74.28 H 6.35 N 4.38 |
| 47 | 4'-$NO_2$ | H | Pale yellow needles | >300 | $C_{15}H_{10}N_2O_5$ | C 61.94 H 3.25 N 9.03<br>C 61.64 H 2.99 N 8.95 |
| 48 | 4'-$NO_2$ | 6-i-$C_3H_7$ | Yellow powder | >300 | $C_{19}H_{15}N_2O_5$ | C 64.77 H 4.58 N 7.95<br>C 64.67 H 4.40 N 7.80 |
| 49 | 4'-$NO_2$ | 6,8-$(Me)_2$ | Yellow powder | >300 | $C_{18}N_{14}N_2O_5$ | C 63.90 H 4.17 N 8.28<br>C 63.93 H 4.11 N 8.21 |
| 50 | 4'-COOMe | H | Colorless powder | >300 | $C_{18}H_{13}NO_5$ | C 66.87 H 4.05 H 4.33<br>C 66.99 H 3.86 N 4.18 |

| Example No. | $R^1$, $R^2$, $R^3$ | $R^4$, $R^5$ | Appearance | M.p. (°C) | Experimental Formula | Elementary Analysis (%) Upper column: Calcd. Lower column: Found |
|---|---|---|---|---|---|---|
| 51 | 4′-COOH | H | 〃 | >300 | $C_{17}H_{11}NO_5$ | C 66.02 H 3.59 N 4.53<br>C 65.77 H 3.38 N 4.25 |
| 52 | 2′,3′,4′-$(OMe)_3$ | 6-Me | Pale yellow crystals | 231~233 | $C_{20}H_{19}NO_6$ | C 65.03 H 5.19 N 3.79<br>C 65.04 H 5.20 N 3.68 |
| 53 | 2′,3′,4′-$(OMe)_3$ | 8-Me | 〃 | 244~246 | $C_{20}H_{19}NO_6$ | C 65.03 H 5.19 N 3.79<br>C 65.21 H 5.13 N 3.63 |
| 54 | 2,3,4-$(OMe)_3$ | 7-$NO_2$ | 〃 | >300 | $C_{19}H_{16}N_2O_8$ | C 57.00 H 4.03 N 7.00<br>C 56.83 H 4.04 N 6.84 |
| 55 (Calcium Salt) | H | 6-n-hexyl | White powder | >300 | $C_{44}H_{44}N_2O_6$ $Ca.H_2O$ | C 70.00 H 6.14 N 3.71<br>C 70.18 H 6.00 N 3.46 |
| 56 | 2,4-Cl | 6-n-hexyl | yellow needles | 294—297° | (hereinafter, results of elementary analysis are omitted.) | |
| 57 | 4-n-pentyl | 6-n-hexyl | yellow flakes | 180—182° | | |
| 58 | 4-Et | 6-n-hexyl | pale yellow needles | 231—233° | | |
| 59 | 4-OMe | 6-n-hexyl | yellow needles | 203—206° | | |
| 60 | H | 6-n-pentyl | yellow needles | 218—219.5° | | |

| Example No. | $R^1$, $R^2$, $R^3$ | $R^4$, $R^5$ | Appearance | M.p. (°C) | Experimental Formula | Elementary Analysis (%) Upper column: Calcd. Lower column: Found |
|---|---|---|---|---|---|---|
| 61 | H | 6-n-octyl | yellow needles | 207—208.5° | | |
| 62 | 4-$NO_2$ | 6-n-hexyl | yellow needles | 264—265° | | |
| 63 | 2-Cl | 6-n-hexyl | yellow needles | 184—185° | | |

**Claims**

1. 4-hydroxy-2-quinolone derivatives represented by the following general formula (I)

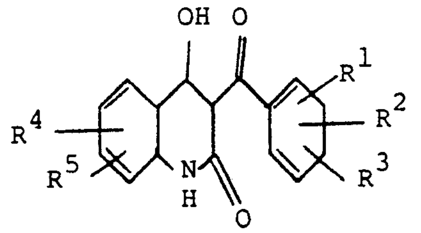

(I)

in which $R^1$, $R^2$, $R^3$, $R^4$ and $R^5$ are same or different and are hydrogen, straight or branched alkyl with one to eight carbon chain length, halogen, straight or branched lower alkoxy with one to five carbon chain length, nitro, cyano, straight or branched halo alkyl with one to three carbon chain length or $COOR^6$ where $R^6$ is hydrogen or straight or branched lower alkyl with one to three carbon chain length; excluding the case where all of $R^1$, $R^2$, $R^3$, $R^4$ and $R^5$ are hydrogen and the case of 3-benzoyl-4-hydroxy-7-methoxy-2-quinolone and pharmaceutically acceptable salts thereof.

2. An anti-inflammatory or anti-allergic composition containing at least one of the compounds represented by the formula (I) as defined in claim 1 excluding the case where all of $R^1$, $R^2$, $R^3$, $R^4$ and $R^5$ are hydrogen, and pharmaceutically acceptable salts thereof, as main ingredient(s).

3. A method of manufacturing a compound of the formula (I) as defined in claim 1 characterized in that a compound of the formula (II)

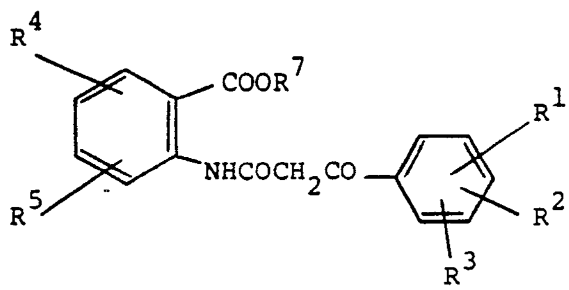

(II)

where $R^1$ to $R^5$ have the same meanings as defined in claim 1; $R_7$ is lower alkyl is subjected to a ring closure reaction.

4. A method of manufacturing a compound of the formula (I) as defined in claim 1, characterized in that a compound of the formula (III)

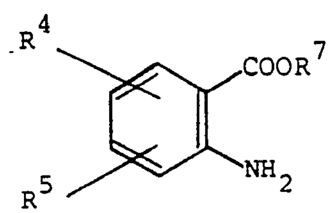

(III)

in which $R^4$, $R^5$ and $R^7$ have the same meanings as defined in claim 1 and claim 3 is treated with a compound of the formula (IV)

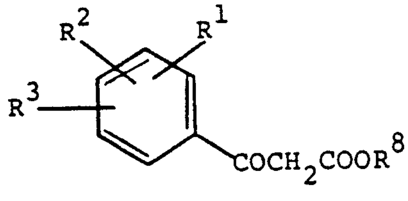

(IV)

in which $R^1$, $R^2$ and $R^3$ have the same meanings as defined in claim 1; $R_8$ is hydrogen or lower alkyl so that a condensation as well as ring closure take place.

5. A method of manufacturing a compound of the formula (I) as defined in claim 1, characterized in that a compound of the formula (V)

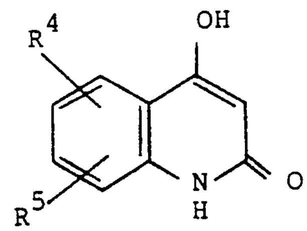

(V)

in which $R^4$ and $R^5$ have the same meanings as defined in claim 1 is subjected to Friedel-Crafts reaction by treating with a compound of the formula (VI)

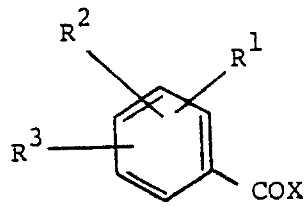

(VI)

(in which $R^1$ to $R^3$ have the same meanings as defined in claim 1; X is halogen).

6. A method of manufacturing a compound of the formula (I) as defined in claim 1, characterized in that a compound of the formula (VII)

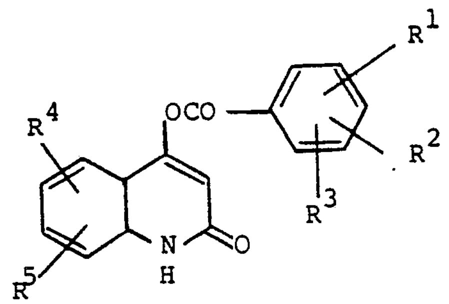

(VII)

in which $R^1$ to $R^5$ have the same meanings as defined in claim 1 is subjected to Fries rearrangement.

7. A compound represented by the formula (I) as defined in claim 1, but excluding the case where all of $R^1$, $R^2$, $R^3$, $R^4$ and $R^5$ are hydrogen, and pharmaceutically acceptable salts thereof, for use as anti-inflammatory or anti-allergic agent.

**Patentansprüche**

1. 4-Hydroxy-2-chinolon-Derivate, dargestellt durch die folgende allgemeine Formel (I)

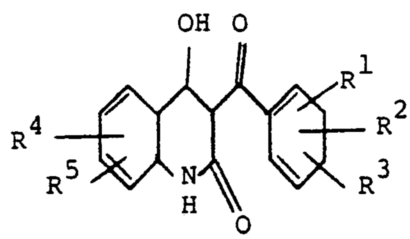

(I)

in der $R^1$, $R^2$, $R^3$, $R^4$ und $R^5$ gleich oder verschieden sind und Wasserstoff, geradkettige oder verzweigte Alkylreste mit einer Kettenlänge von 1 bis 8 Kohlenstoffatomen, Halogen, geradkettige oder verzweigte Alkoxyreste mit einer Kettenlänge von 1 bis 5 Kohlenstoffatomen, Nitro, Cyano, geradkettige oder verzweigte halogenierte Alkylreste mit einer Kettenlänge von 1 bis 3 Kohlenstoffatomen oder $COOR^6$ bedeuten, worin $R^6$ Wasserstoff oder ein geradkettiger oder verzweigter Alkylrest mit einer Kettenlänge von 1 bis 3 Kohlenstoffatomen ist, wobei ausgenommen sind der Fall, in dem alle Reste $R^1$, $R^2$, $R^3$, $R^4$ und $R^5$ Wasserstoff bedeuten, und der Fall des 3-Benzoyl-4-hydroxy-7-methoxy-2-chinolons, und deren pharmazeutisch annehmbare Salze.

2. Eine entzündungshemmende oder antiallergische Zusammensetzung, enthaltend wenigstens eine der Verbindungen der in Anspruch 1 definierten Formel (I) und deren pharmazeutisch annehmbare Salze als Hauptkomponente(n), ausgenommen der Fall, in dem alle Reste $R^1$, $R^2$, $R^3$, $R^4$ und $R^5$ Wasserstoff bedeuten.

3. Verfahren zur Herstellung einer Verbindung der in Anspruch 1 definierten Formel (I), dadurch gekennzeichnet, daß eine Verbindung der Formel (II)

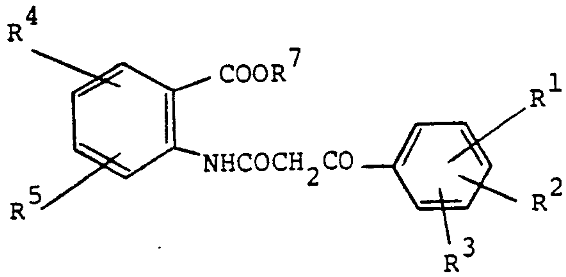

(II)

in der $R^1$ bis $R^5$ die gleichen Bedeutungen wie in Anspruch 1 haben und $R_7$ ein niederer Alkylrest ist, einer Ringschlußreaktion unterworfen wird.

4. Verfahren zur Herstellung einer Verbindung der in Anspruch 1 definierten Formel (I), dadurch gekennzeichnet, daß eine Verbindung der Formel (III)

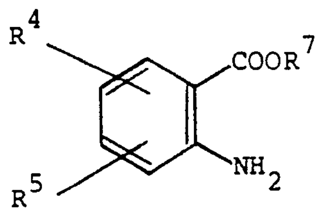

(III)

in der $R^4$, $R^5$ und $R_7$ die gleichen Bedeutungen wie in den Ansprüchen 1 und 3 haben, mit einer Verbindung der Formel (IV) behandelt wird,

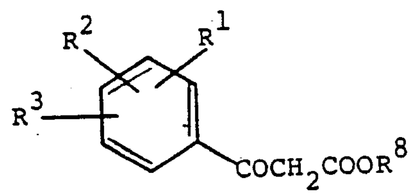

(IV)

in der $R^1$, $R^2$ und $R^3$ die gleichen Bedeutungen wie im Anspruch 1 haben und $R^8$ Wasserstoff oder niederes Alkyl bedeutet, so daß eine Kondensationsreaktion und eine Ringschlußreaktion stattfindet.

5. Verfahren zur Herstellung einer Verbindung der in Anspruch 1 definierten Formel (I), dadurch gekennzeichnet, daß eine Verbindung der Formel (V)

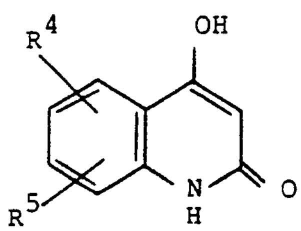

(V)

in der $R^4$ und $R^5$ die gleichen Bedeutungen haben wie in Anspruch 1, einer Friedel-Crafts-Reaktion durch Behandlung mit einer Verbindung der Formel (VI) unterworfen wird,

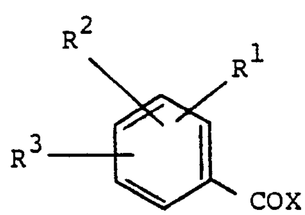

(VI)

in der $R^1$ bis $R^3$ die gleichen Bedeutungen haben wie im Anspruch 1 und X eine Halogen ist.

6. Verfahren zur Herstellung einer Verbindung der in Anspruch 1 definierten Formel (I), dadurch gekennzeichnet, daß eine Verbindung der Formel (VII)

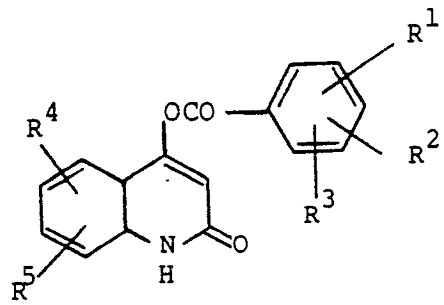

(VII)

in der $R^1$ bis $R^5$ die gleichen Bedeutungen wie in Anspruch 1 haben, einer Fries-Umlagerung unterworfen wird.

7. Eine Verbindung dargestellt durch die in Anspruch 1 definierte Formel (I), ausgenommen der Fall, in dem alle Reste $R^1$, $R^2$, $R^3$, $R^4$ und $R^5$ Wasserstoff bedeuten, und deren pharmazeutisch annehmbare Salze, zur Verwendung als entzündungshemmendes oder antiallergisches Mittel.

**Revendications**

1. Dérivés de la 4-hydroxy-2-quinolone représentés par la formule générale (I) suivante:

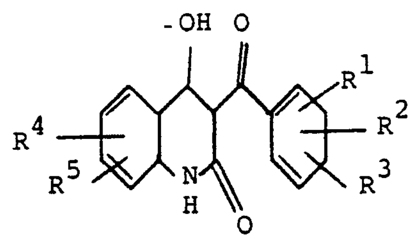

(I)

(dans laquelle $R^1$, $R^2$, $R^3$, $R^4$ et $R^5$ sont identiques ou differents et sont un atome d'hydrogène, un groupe alkyle linéaire ou ramifié en $C_2$ à $C_8$, un atome d'halogène, un groupe alcoxy inférieur linéaire ou ramifié en $C_1$ à $_5$, un groupe nitro, cyano, un groupe haloalkyle linéaire ou ramifie en $C_1$ à $C_3$ ou $COOR^6$ où $R^6$ est un atome d'hydrogène ou un groupe alkyle inférieur linéaire ou ramifié en $C_1$ à $C_3$; à l'exclusion du cas où tous les symboles $R^1$, $R^2$, $R^3$, $R^4$ et $R^5$ sont des atomes d'hydrogène et du cas de la 3-benzoyl-4-hydroxy-7-méthoxy-2-quinolone, et leurs sels pharmaceutiquement acceptables.

2. Composition anti-inflammatoire ou anti-allergique contenant au moins un des composés représentés par la formule (I) tel que définis dans la revendication 1, à l'exclusion du cas où tous les symboles $R^1$, $R^2$, $R^3$, $R^4$ et $R^5$ sont des atomes d'hydrogène, et leurs sels pharmaceutiquement acceptables, comme ingrédients actifs.

3. Procédé de préparation d'un composé répondant à la formule (I) selon la revendication 1, caractérisé en ce qu'on soumet un composé répondant à la formule (II)

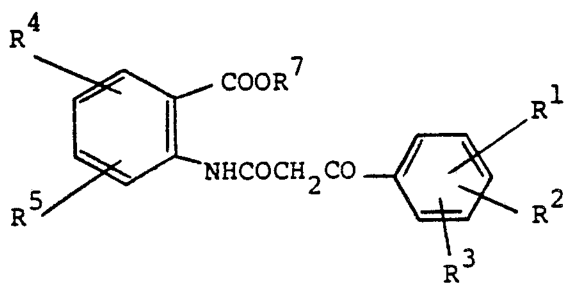

(II)

dans laquelle $R^1$ à $R^5$ ont les mêmes significations que dans la revendication 1, $R_7$ est un groupe alkyle inférieur, à une réaction de fermeture de cycle.

4. Procédé de préparation d'un composé répondant la formule (I) selon la revendication 1, caractérisé en ce qu'on traite un composé répondant à la formule (III)

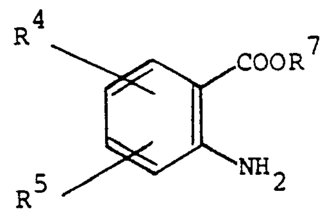

(III)

dans laquelle $R^4$, $R^5$ et $R^7$ ont les mêmes significations que dans la revendication 1 et la revendication 3, par un composé répondant à la formula (IV)

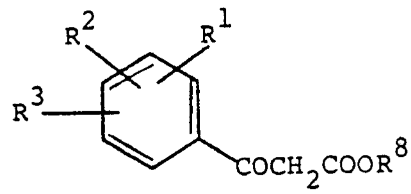

(IV)

dans laquelle $R^1$, $R^2$ et $R^3$ ont les mêmes significations que dans la revendication; $R^8$ est un atome d'hydrogène ou un groupe alkyle inférieur, de telle sorte qu'il se produit une condensation ainsi qu'une fermeture de cycle.

5. Procédé de préparation d'un composé répondant à la formule (I) selon la revendication 1, caractérisé en ce qu'on soumet un composé répondant à la formule (V)

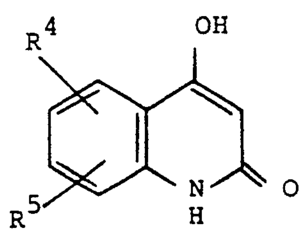

(V)

dans laquelle $R^4$ et $R^5$ ont les mêmes significations que dans la revendication 1, à une réaction de Friedel-Crafts en le traitant par un composé répondant à la formule (VI)

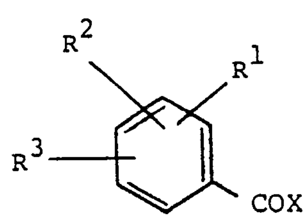

(VI)

(dans laquelle $R^1$ à $R^3$ ont les mêmes significations que dans la revendication 1; X est un atome d'halogène).

6. Procédé de préparation d'un composé répondant à la formule (I) selon la revendication 1, caractérisé en ce qu'on soumet un composé répondant à la formule (VII)

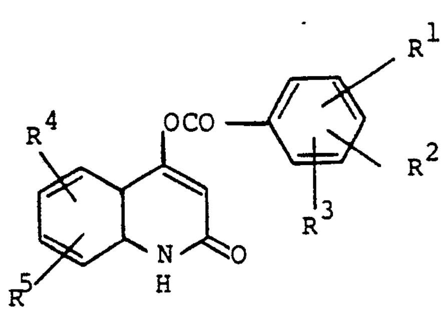

(VII)

dans laquelle $R^1$ à $R^5$ ont les mêmes significations que dans la revendication 1, à un réarrangement de Fries.

7. Composé représenté par la formule (I) tel que défini dans la revendication 1, mais à lexclusion du cas où tous les symboles $R^1$, $R^2$, $R^3$, $R^4$ et $R^5$ sont des atomes d'hydrogène, et leurs sels pharmaceutiquement acceptables pour l'utilisation comme agents anti-inflammatoire ou anti-allergique.